# EUROPEAN PATENT APPLICATION

(11) **EP 4 674 389 A1**
(43) Date of publication of application: **07.01.2026**
(21) Application number: 24306108.2
(22) Date of filing: 04.07.2024
(51) Int. Cl.: A61F 2/954, A61F 2/07

(54) **DEVICE TO FACILITATE CANNULATION OF AORTIC ENDOPROSTHESIS CONTRALATERAL LIMB**

(71) Applicant: Université de Strasbourg, 67000 Strasbourg (FR); Hôpitaux Universitaires de Strasbourg (HUS), 67000 Strasbourg (FR)
(72) Inventor: CHAKFÉ, Nabil, 67150 Hindisheim (FR); LACHEGUR, Wissal, 67100 Strasbourg (FR)
(74) Representative: IPAZ

(57) **Abstract**

Device (20) intended to position a guiding element, the latter being intended to guide a first delivery system designed to release a contralateral limb of an aortic endoprosthesis. The device (20) comprises a coupling mechanism (22), like a gripper, configured to cooperate with a sheath (70) of a homolateral delivery system (62) to guide device (20) while it is moved towards a main body (11) of endoprosthesis.

## Description

### Technical field

The present invention relates to vascular surgery devices and techniques.

The invention is of particular interest for installing modular bifurcated aortic endoprostheses.

### Background

Vascular endoprostheses, also known as "stent grafts" or "cover stents", are commonly used to create new blood passageways within human blood vessels, notably for treating abdominal aortic aneurysms.

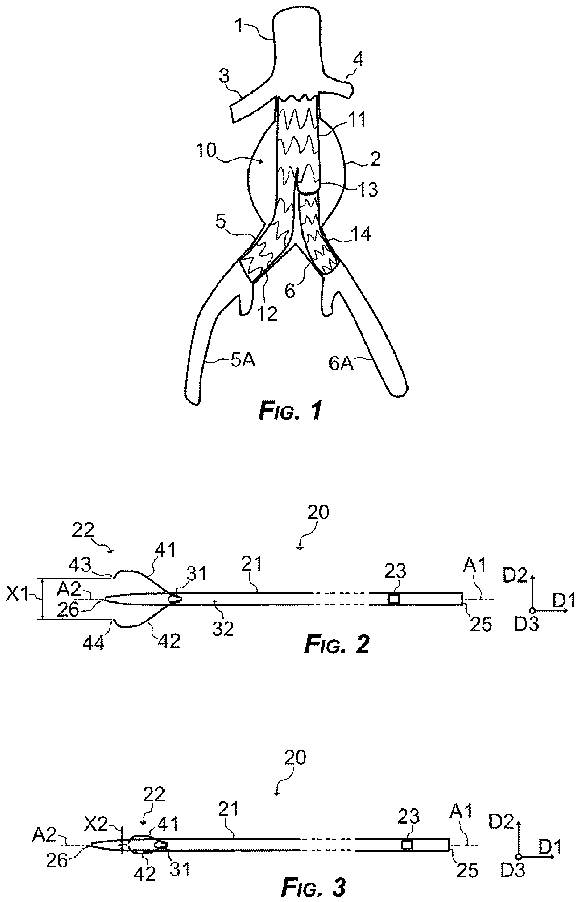

Figure 1 diagrammatically shows a part of a human arterial system including abdominal aorta 1 with aneurysm 2, renal arteries 3 and 4, and right and left common iliac arteries 5 and 6, respectively. A conventional modular bifurcated aortic endoprosthesis 10 is arranged in this arterial system to bypass the aneurysm 2. Endoprosthesis 10 comprises a first module having a main body 11 located within aorta 1, a homolateral limb 12 extending into the right common iliac artery 5, and a short limb 13 facing the left common iliac artery 6. A second module is connected to short limb 13 and extends into left common iliac artery 6 to form a contralateral limb 14 of endoprosthesis 10.

As known per se, the first, respectively the second, module of such an endoprosthesis 10 can be endoluminally inserted through an incision within common femoral artery 5A, respectively 6A. After deployment of main body 11, contralateral common iliac artery 6 is cannulated to join the contralateral limb 14 to the short limb 13 of the first module.

Such a cannulation is critical and often challenging, especially for surgeons with little expertise, particularly when the arterial system comprises iliac tortuosities, and/or a large aneurysm with little thrombosis leaving a large space around the first module of the endoprosthesis. This can significantly impact the procedure's overall duration and radiation exposure.

US6676694B1 discloses a technique for installing a bifurcated aortic endoprosthesis using catheters with magnets. Among other drawbacks, this technique requires complete deployment of the homolateral limb before contralateral cannulation.

US8821567B2 discloses another technique for installing a bifurcated aortic endoprosthesis using an escort catheter having a snare wire to move the wire of a contralateral catheter. This technique requires increasing the diameter of, or incorporating the escort catheter to, the homolateral delivery system. In addition, escort catheter is moved along the axis of the homolateral delivery system, which can be misdirected.

### Description of the invention

To overcome the limitations of the prior art, the invention provides a device intended to position a guiding element, this guiding element being intended to guide a first delivery system designed to release a contralateral limb of an aortic endoprosthesis.

The device comprises a body and a coupling mechanism, said body having an opening intended to receive said guiding element, said coupling mechanism comprising one or more legs having a free distal end.

According to the invention, the device is configured to allow a change of configuration of said coupling mechanism between:
- a first configuration in which said one or more legs are configured to allow the coupling mechanism to engage with a homolateral structure,
- a second configuration in which said one or more legs are configured to cooperate with said homolateral structure to guide the device while it is moved towards a main body of said endoprosthesis.

Preferably, said homolateral structure may comprise a sheath of a second delivery system designed to release a homolateral limb of said endoprosthesis. As another non-limiting example, said homolateral structure may comprise said homolateral limb of said endoprosthesis, preferably after deployment.

The device of the invention is thus configured to be able to use said homolateral structure, e.g. said second delivery system, as a guiding means, improving accuracy and duration of positioning the device, which can then be used to install a guiding element designed to guide said first delivery system.

The invention can significantly reduce contralateral limb cannulation duration on radiation exposure during standard endovascular aneurysm repair procedures.

In addition, the device of the invention does not need to be integrated with said second delivery system and is advantageously compatible with delivery systems available in the market.

As a further advantage, the invention avoids the use of several catheters of different shapes when cannulation is difficult, reducing costs and procedure duration.

In one embodiment, the device is configured so that:
- in said first configuration, the distal end of a first of said legs and the distal end of a second of said legs are distant from one another to allow the coupling mechanism to engage with said homolateral structure,
- in said second configuration, the distal end of said first leg and the distal end of said second leg are close to each other to allow the coupling mechanism to cooperate with said homolateral structure.

In other words, said coupling mechanism may form a gripper.

Of course, the coupling mechanism is configured to cooperate with said homolateral structure in a relatively loose manner to allow the device to move relative to the homolateral guiding structure while providing the guidance function.

In one embodiment, said one or more legs are configured to form at least one loop wrapping around said homolateral structure when the coupling mechanism is in the second configuration.

For example, in the embodiment described above where the coupling mechanism comprises said first leg and said second leg, these first and second legs may form together one loop in said second configuration.

According to another example, the coupling mechanism may comprise one leg forming a loop in said second configuration.

In one embodiment, the body of the device is configured to guide said one or more legs of the coupling mechanism as it is moved from one to another of said configurations.

In one embodiment, said opening of the body is a first opening, the body comprising a second opening that receives the coupling mechanism.

Preferably, said second opening opens onto a lateral external surface of the body.

In one embodiment, the device is configured to allow the coupling mechanism to be placed in a third configuration in which the legs are housed in said second opening.

In one embodiment, the coupling mechanism comprises one or more wires forming said one or more legs.

In one embodiment, the device comprises a control element configured to move the coupling mechanism from one to another of said configurations.

In one embodiment, said control element comprises a cursor or a thumbwheel.

Preferably, the control element is connected to the body of the device.

In one embodiment, said control element is connected to a proximal end of the coupling mechanism.

For example, in the embodiment described above where the coupling mechanism comprises one or more wires forming said one or more legs, said control element may be connected to a proximal end of one or more of said wires.

In one embodiment, the body of the device comprises a main portion extending along a longitudinal axis and a distal portion curved with respect to said main portion and/or extending along an oblique axis with respect to said longitudinal axis.

In one embodiment, said one or more legs are configured to extend obliquely with respect to the body of the device when the coupling mechanism is in said first configuration and/or in said second configuration.

In one embodiment, the body of the device comprises a biocompatible material, for example a biocompatible polymer material.

In one embodiment, the coupling mechanism comprises a memory material.

In one embodiment, the coupling mechanism comprises a biocompatible material, for example nickel-titanium alloy, or stainless steel.

Yet another object of the invention is to provide an equipment comprising such a device and at least one guiding element, said opening of the body of the device being configured to receive said guiding element.

In one embodiment, said at least one guiding element comprises at least one wire.

In one embodiment, said guiding element comprises a biocompatible material, for example a biocompatible polymer material or metallic alloy.

The invention also provides a method for installing a vascular endoprosthesis, using a device and/or an equipment as defined above.

In one mode of operation, the method comprises:
- inserting the device into a first, e.g. left, common femoral artery of a human arterial system,
- engaging the coupling mechanism with a homolateral structure, for example a delivery system, previously inserted into a second, e.g. right, common femoral artery of said arterial system, and
- moving the device using said homolateral structure as a guide thanks to cooperation of said coupling mechanism with this homolateral structure.

In one mode of operation, the method comprises inserting a guiding element in an opening of the device, and using this guiding element to guide the device so that a portion of the device is displaced through a deployed part, e.g. the main body, of endoprosthesis.

In one mode of operation, the method comprises deploying and preferably rotating the coupling mechanism to check whether the device is correctly located within said deployed part, e.g. the main body, of endoprosthesis.

In one mode of operation, the method comprises:
- replacing said guiding element with a second guiding element,
- removing said device, and
- using said second guiding element to position another delivery system into said first common femoral artery.

The present invention, and some of its features and advantages, will become more readily apparent in view of the detailed description of particular embodiments provided below, including the accompanying drawings.

### Brief description of the drawings

In the following, non-limiting embodiments of the invention are described with reference to the accompanying drawings in which:
- Figure 1, described above, is a schematic representation of an aortic endoprosthesis arranged within a human arterial system;
- Figure 2 is a schematic representation, in front view, of a device according to the invention, the device comprising a coupling mechanism, forming in this example a gripper, shown in a first configuration;
- Figure 3 is a schematic representation, in front view, of the device of figure 2 showing the coupling mechanism in a second configuration;
- Figure 4 is a schematic representation, in front view, of the device of figure 2 showing the coupling mechanism in another configuration;
- Figure 5 is a schematic representation, in side view, of the device of figure 2, the coupling mechanism being in a configuration similar to that shown in figure 3;
- Figure 6-19 schematically illustrate a succession of steps for installing an endoprosthesis in the configuration shown in figure 1:
   ∘ Figure 6 depicts insertion of a delivery system for deploying a first module of the endoprosthesis, this delivery system having a sheath;
   ∘ Figure 7 depicts partial deployment of the endoprosthesis first module, more specifically of main body and short limb of endoprosthesis;
   ∘ Figure 8 depicts insertion of a device according to the invention;
   ∘ Figure 9 depicts cooperation of the device with the delivery system sheath, with a cooperation mechanism of the device;
   ∘ Figure 10 depicts positioning a distal portion of the device in front of the endoprosthesis short limb;
   ∘ Figure 11 depicts insertion of a guiding wire in an opening of the device;
   ∘ Figure 12 depicts retraction of said cooperation mechanism;
   ∘ Figure 13 depicts insertion of the device in the endoprosthesis main body, and deployment of the coupling mechanism;
   ∘ Figure 14 depicts positioning the device through the endoprosthesis main body;
   ∘ Figure 15 depicts removing the guiding wire from the device;
   ∘ Figure 16 depicts insertion of a second guiding wire in the device opening;
   ∘ Figure 17 depicts removing the device from the arterial system;
   ∘ Figure 18 depicts insertion of another delivery system for deploying a second module of the endoprosthesis;
   ∘ Figure 19 depicts deployment of the endoprosthesis second module, forming a contralateral limb.

### Detailed description of embodiments

Figures 2 to 5 illustrate schematically a device 20 according to a non-limiting embodiment of the invention.

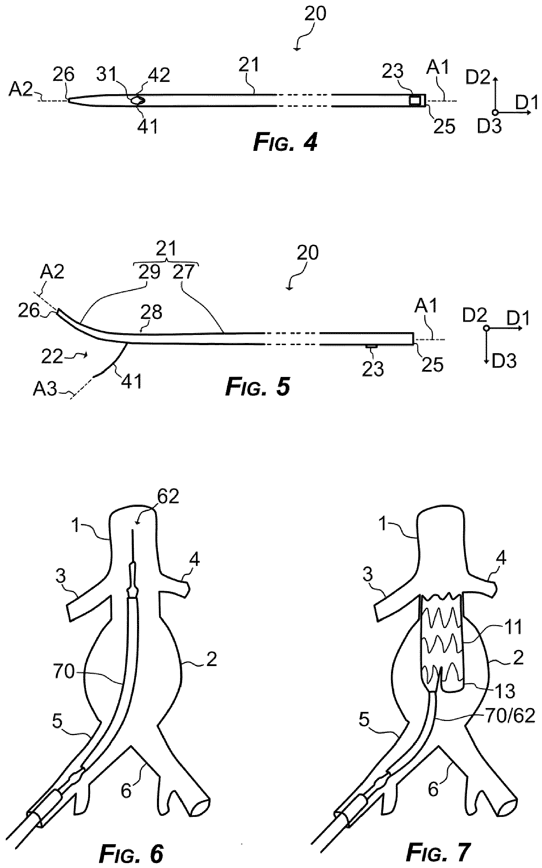

The device 20 is designed for installing a vascular endoprosthesis, for example as described below with reference to figures 6 to 19.

In the embodiment of figures 2 to 5, device 20 comprises a body 21, a gripper 22 forming a coupling mechanism and a cursor 23 forming a control element.

Figures 2 to 5 provide an orthogonal spatial system formed by directions D1, D2 and D3 associated with the body 21 of device 20.

Body 21 globally has an elongated shape along direction D1, forming a proximal end 25 and a distal end 26.

With reference to figure 5, body 21 has in this non-limiting example a main portion 27 extending from proximal end 25 to an inflection point 28, and a distal portion 29 extending from inflection point 28 to distal end 26.

The main portion 27 extends along a fictitious longitudinal axis A1 parallel to D1.

The distal portion 29 of body 21 has a curved shape extending globally along a fictitious longitudinal axis A2 which is oblique to axis A1. Referring to figures 2 and 5, axis A1 and A2 are both parallel to a fictitious plane D1-D3.

Body 21 has a first opening (not shown) extending longitudinally to open out at proximal end 25 and at distal end 26. Said first opening is designed to receive a guiding wire (see below).

In this example, body 21 further comprises a second opening (not shown) extending longitudinally from control element 23 - in particular when control element 23 is in a position corresponding to a so-called retracted configuration of coupling mechanism (see below) - to a lateral aperture 31 through which said second opening ends in a lateral external surface 32 of body 21.

In this embodiment, said first opening is a central opening, i.e. centred on axis A1 in main portion 27 of body 21 and on axis A2 in said distal portion 29, and said second opening extends radially between the central opening and external surface 32 of body 21.

In this non-limiting example, body 21 comprises a biocompatible material, preferably a biocompatible polymer material.

In the embodiment of figures 2-5, coupling mechanism 22 comprises two wires 41 and 42, each having a proximal end (not shown) and a distal end 43 and 44, respectively.

Wires 41 and 42 are received in said second opening of body 21, with their proximal end connected to the control element 23.

In configurations shown in figures 2 to 5, respective portions of the wires 41 and 42 extend through aperture 31, so that distal ends 43 and 44 extend outside the body 21.

Wires 41 and 42 thus form two respective legs with said distal ends 43 and 44 free.

Figure 2 illustrates a first configuration - also called deployed configuration, or approach configuration, or engaging configuration - in which legs 41 and 42 are maximally deployed. In this configuration, distal ends 43 and 44 are spaced from each other by a distance X1 in direction D2.

Figure 3 illustrates a second configuration, also called gripping configuration, in which distal ends 43 and 44 are spaced from each other by a distance X2 in direction D2. X2 is smaller than X1 and can be zero.

In said retracted configuration (not shown), legs 41 and 42 are fully housed in the second opening of body 21.

Figure 4 illustrates a third, intermediate, configuration, in which a small portion of legs 41 and 42 extends outside the body 21.

In this embodiment, legs 41 and 42 comprises a memory material, in this example nickel-titanium alloy also referred to as nitinol, to take a predefined shape when legs 41 and 42 are moved from retracted configuration to configurations of figures 2 or 3.

In the configurations of figures 2 and 3, legs 41 and 42 each have a curved shape so that distal ends 43 and 44 are arranged facing each other.

In the configuration of figure 2, legs 41 and 42 form together a loop.

Referring to the configuration of figure 4, legs 41 and 42 extend in this example along a fictitious axis A3 which is oblique to both axis A1 and A2, and parallel to the plane D1-D3. In this configuration, A2 is directed to one side in direction D3, while A3 is directed to the opposite side in direction D3.

In this embodiment, the change of configuration of the gripper 22 formed by wires 41 and 42 can be controlled by the control element 23, here a cursor.

In this example, cursor 23 is connected to body 21 by a sliding connection, so that a movement of cursor 23 in direction D1 results in a corresponding movement of wires 41 and 42, moving the gripper 22 from one configuration to another.

Legs 41 and 42 of gripper 22 are in this non-limiting example guided by a portion of the body 21 delimiting aperture 31 when gripper 22 is moved between said retracted configuration and deployed configuration.

It will now be described a non-limiting method for installing a vascular endoprosthesis using a device according to the invention, for example device 20 according to the embodiment described above.

In this particular example, the method is carried out to install a standard modular bifurcated aortic endoprosthesis as illustrated in figure 1.

The method comprises a non-limiting succession of steps described below with reference to figures 6 to 19. Some of these steps being known per se, the following description is not exhaustive and is intended mainly to illustrate advantages of the invention and specific innovative steps.

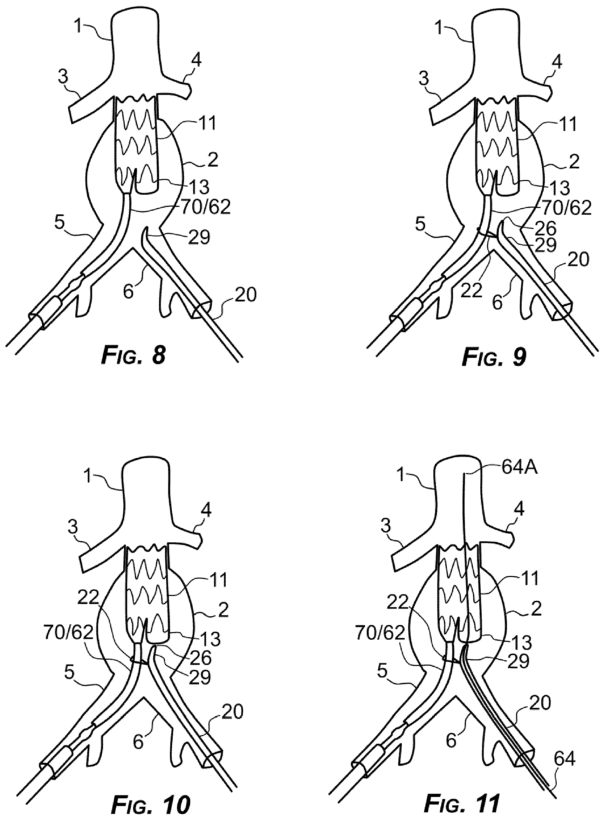

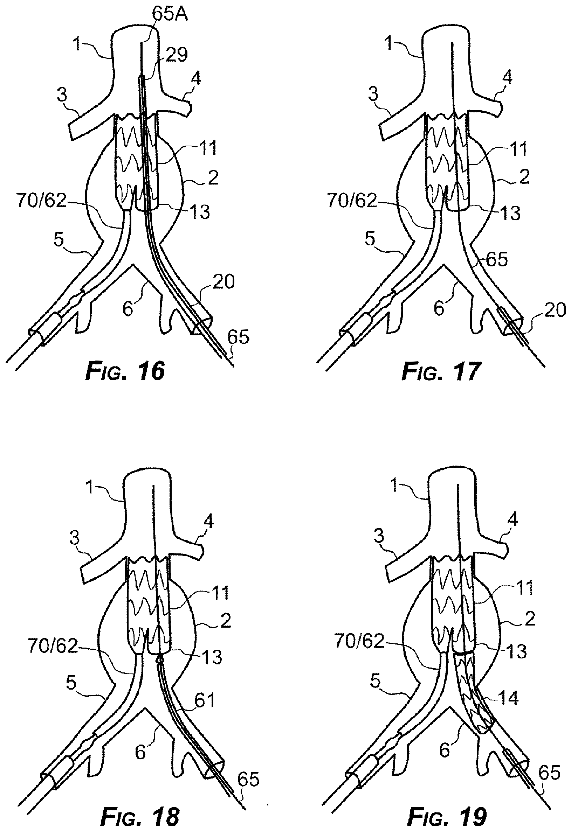

In this example, the method uses an equipment that non-limitatively includes, in addition to the inventive device 20, a first delivery system 61 (see figure 18), a second delivery system 62 (see figures 6-19), a first guiding element 64 (see figures 11-15), and a second guiding element 65 (figures 16-19).

Referring to figure 6, delivery system 62 is inserted into the right common iliac artery 5 in the classic way so that a sheath 70 of system 62 extends through the aneurysm cavity 2 of artery 1.

The first module of endoprosthesis 10 is partially deployed with the delivery system 62 as shown in figure 7. More specifically, at this stage, the endoprosthesis main body 11 and short limb 13 are deployed, homolateral limb remaining inside the sheath 70.

Device 20 is then inserted into the left common iliac artery 6 until its distal portion 29 enters the cavity 2. During this step, illustrated in figure 8, the coupling mechanism 22 is kept retracted.

The distal portion 29 of device 20 is positioned beside sheath 70 and coupling mechanism 22 is moved from retracted configuration to said first configuration (see e.g. figure 2), to engage with sheath 70. Coupling mechanism 22 is then moved from said first configuration to said second configuration (see e.g. figure 3), to cooperate with sheath 70 as illustrated in figure 9.

Referring to figures 9 and 10, device 20 is then moved towards endoprosthesis short limb 13 and main body 11, sheath 70 forming a guide means.

After positioning the device distal end 26 in front of aperture of the endoprosthesis short limb 13, a wire forming said first guiding element 64 is inserted through said first opening of device 20 until a distal end 64A of wire 64 extends into aorta 1 beyond the endoprosthesis main body 11 (see figure 11). In this example, wire 64 comprises a metal alloy.

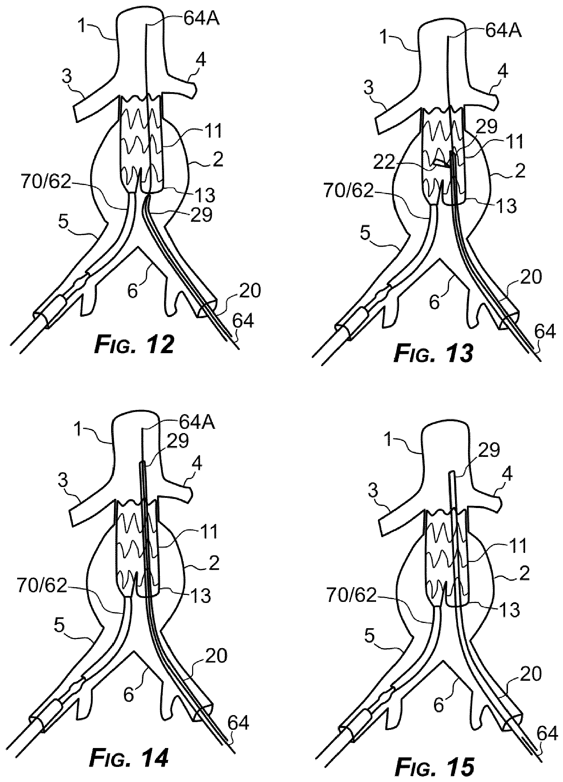

From the configuration of figure 11, coupling mechanism 22 is retracted as shown in figure 12 and device 20 is moved through the endoprosthesis short limb 13 so that the device distal portion 29 extends inside the endoprosthesis main body 11.

Referring to figure 13, coupling mechanism 22 is then totally or partially deployed, and optionally the body of device 20 is rotated about its axis, to ensure that device 20 is correctly positioned. Indeed, if the device distal portion 29 is positioned outside the endoprosthesis main body 11, in particular radially between the endoprosthesis main body 11 and the wall of aorta 1, coupling mechanism 22 could not be deployed and/or body of device 20 could not be rotated as coupling mechanism 22 is deployed.

With the coupling mechanism 22 retracted, device 20 is moved through the endoprosthesis main body 11 so that the device distal portion 29 extends in aorta 1 beyond the endoprosthesis main body 11, as illustrated in figure 14.

Wire 64 is then withdrawn from device 20 (see figure 15) and another wire 65, forming said second guiding element, is then inserted through said first opening of device 20 until a distal end 65A of wire 65 extends into aorta 1 beyond the endoprosthesis main body 11, as shown in figure 16.

In this example, wire 65 comprises a metal alloy and is configured to be more rigid than wire 64.

Device 20 is then withdrawn as illustrated in figure 17.

Referring to figures 18 and 19, delivery system 61 is inserted using the guiding wire 65 to release the contralateral limb 14 forming said second module of endoprosthesis 10.

In this example, delivery system 62 is then operated to release the endoprosthesis homolateral limb 12, to reach the configuration of figure 1.

As known per se, imaging technique such as fluoroscopy can be used for real-time monitoring of endoprosthesis installation.

Many variations can be made to the above-described device and technique.

For example, in one embodiment, not shown, the coupling mechanism of the inventive device may comprise a unique leg configured to be moved from a configuration allowing engagement with sheath 70, or more generally with a homolateral guiding structure, to a configuration in which said leg cooperate with said homolateral structure to guide the device as described above. For example, the leg of coupling mechanism may be formed by a wire having a memory material such as nitinol to wrap around the homolateral structure when the coupling mechanism is deployed. The above description applies mutatis mutandis to such an embodiment.

As non-limiting alternatives of the invention, the device control element may comprise a thumbwheel or any other suitable means, or may be formed by portion and/or proximal end of the one or more legs.

Of course, the one or more legs of coupling mechanism may comprise another material than nitinol, for example stainless steel.

More generally, the material and/or the shape of the components of the inventive device and/or of the described equipment may be different from those indicated above without departing from the scope of the invention.

## Claims

1. Device (20) intended to position a guiding element (65) which is intended to guide a first delivery system (61) designed to release a contralateral limb (14) of an aortic endoprosthesis (10), comprising a body (21) and a coupling mechanism (22), said body (21) having an opening intended to receive said guiding element (65), said coupling mechanism (22) comprising one or more legs (41, 42) having a free distal end (43, 44), said device (20) being configured to allow a change of configuration of said coupling mechanism (22) between:
- a first configuration in which said one or more legs (41, 42) are configured to allow the coupling mechanism (22) to engage with a homolateral structure, said homolateral structure being preferably a sheath (70) of a second delivery system (62) designed to release a homolateral limb (12) of said endoprosthesis (10),
- a second configuration in which said one or more legs (41, 42) are configured to cooperate with said homolateral structure (70) to guide the device (20) while it is moved towards a main body (11) of said endoprosthesis (10).

2. Device (20) according to claim 1, wherein:
- in said first configuration, the distal end (43) of a first of said legs (41) and the distal end (44) of a second of said legs (42) are distant (X1) from one another to allow the coupling mechanism (22) to engage with said homolateral structure (70),
- in said second configuration, the distal end (43) of said first leg (41) and the distal end (44) of said second leg (42) are close (X2) to each other to allow the coupling mechanism (22) to cooperate with said homolateral structure (70).

3. Device (20) according to claim 1 or 2, wherein said one or more legs (41, 42) are configured to form at least one loop wrapping around said homolateral structure (70) when the coupling mechanism (22) is in the second configuration.

4. Device (20) according to anyone of claims 1 to 3, wherein the body (21) of the device (20) is configured to guide said one or more legs (41, 42) of the coupling mechanism (22) as it is moved from one to another of said configurations.

5. Device (20) according to anyone of claims 1 to 4, wherein said opening of the body (21) is a first opening, the body (21) comprising a second opening that receives the coupling mechanism (22) and opens (31) onto a lateral external surface (32) of the body (21).

6. Device (20) according to claim 5, configured to allow the coupling mechanism (22) to be placed in a third configuration in which the legs (41, 42) are housed in said second opening.

7. Device (20) according to anyone of claims 1 to 6, wherein the coupling mechanism (22) comprises one or more wires forming said one or more legs (41, 42).

8. Device (20) according to anyone of claims 1 to 7, comprising a control element (23) configured to move the coupling mechanism (22) from one to another of said configurations.

9. Device (20) according to claim 8, wherein said control element (23) comprises a cursor or a thumbwheel, the control element (23) being preferably connected to the body (21) of the device (20).

10. Device (20) according to anyone of claims 1 to 9, wherein said control element (23) is connected to a proximal end of the coupling mechanism (22).

11. Device (20) according to anyone of claims 1 to 10, wherein the body (21) of the device (20) comprises a main portion (27) extending along a longitudinal axis (A1) and a distal portion (29) curved with respect to said main portion (27) and/or extending along an oblique axis (A2) with respect to said longitudinal axis (A1).

12. Device (20) according to anyone of claims 1 to 11, wherein said one or more legs (41, 42) are configured to extend obliquely with respect to the body (21) of the device (20) when the coupling mechanism (22) is in said first configuration and/or in said second configuration.

13. Equipment comprising a device (20) according to anyone of claims 1 to 12 and at least one guiding element (64, 65), said opening of the body (21) of the device (20) being configured to receive said guiding element (64, 65).

14. Equipment according to claim 13, wherein said at least one guiding element (64, 65) comprises at least one wire.
